# EUROPEAN PATENT APPLICATION

(11) **EP 4 089 682 A1**
(43) Date of publication of application: **16.11.2022**
(21) Application number: 21173445.4
(22) Date of filing: 12.05.2021
(51) Int. Cl.: G16H 10/20, A61B 5/00, G16H 20/30, G16H 20/70, G16H 40/63, G16H 50/20, A61B 7/00, G10L 17/26, G10L 25/27, G10L 25/66

(54) **MEDICAL SUPPORT SYSTEM AND MEDICAL SUPPORT METHOD FOR PATIENT TREATMENT**

(71) Applicant: BIOTRONIK SE & Co. KG, 12359 Berlin (DE)
(72) Inventor: DÖRR, Thomas, 12437 Berlin (DE); MÜLLER, Jens, 14195 Berlin (DE); GRATZ, Matthias, 91054 Erlangen (DE); WHITTINGTON, R. Hollis, Portland, 97202 (US)
(74) Representative: Biotronik Corporate Services SE

(57) **Abstract**

The present disclosure provides a medical support system (100) for patient treatment. The medical support system (100) includes an audio reception module (110) configured to receive sounds (S) of a patient (1) and generate sound data (SD) indicative of the received sounds (S); and an artificial intelligence module (120) configured to analyze the sound data (SD), wherein the artificial intelligence module (120) is further configured to provide a medical support function based on the analysis of the sound data (SD).

## Description

Embodiments of the present disclosure relate to a medical support system for patient treatment, a medical support method for patient treatment, and a machine readable medium to execute the medical support method. Embodiments of the present disclosure relate particularly to a non-invasive, automated progress monitoring and/or therapy control applicable to a broad patient population.

In today's healthcare delivery, for example in hospitals and through ambulance services, it is increasingly difficult to find the optimal treatment for a patient. In particular, there is an increasing challenge to identify the most effective treatment for a patient as well as to reduce costs and achieve greater efficiency. These difficulties are due, at least in part, to rising cost pressures, an increasing number of treatment options, and increased information availability. It is therefore desirable to identify optimal treatments, minimize a treatment time and reduce medical costs.

In light of the above, a medical support system for patient treatment, a medical support method for patient treatment, and a machine readable medium to execute the medical support method are provided.

It is an object of the present disclosure to improve identification of a patient condition. It is another object of the present disclosure to minimize a treatment time and/or reduce medical costs.

The objects are solved by the features of the independent claims. Preferred embodiments are defined in the dependent claims.

According to an independent aspect of the present disclosure, a medical support system for patient treatment is provided. The medical support system includes an audio reception module configured to receive sounds, such as speech, of a patient and generate sound data indicative of the received sounds; and an artificial intelligence module configured to analyze the sound data, wherein the artificial intelligence module is further configured to provide a medical support function for the patient based on the analysis of the sound data.

According to some embodiments, which can be combined with other embodiments described herein, the artificial intelligence module may be configured to implement a machine learning algorithm.

The term "machine learning algorithm" as used throughout the present application refers to a software algorithm that can build a model based on training data, in order to make predictions and/or decisions without being explicitly programmed to do so.

Preferably, the artificial intelligence module may implement a neural network to analyse at least the sound data in order to provide the medical support function for the patient.

In the context of the present disclosure, the neural network may be suitably trained to process the sound data and optionally other health-related patient data to provide the medical support function.

According to some embodiments, which can be combined with other embodiments described herein, the artificial intelligence module may be configured to determine at least one vocal biomarker in the sound data. The artificial intelligence module may be configured to provide the medical support function based on the determined at least one vocal biomarker.

According to some embodiments, which can be combined with other embodiments described herein, the artificial intelligence module may be configured to provide the medical support function further based on one or more health condition parameters of the patient.

In a preferred embodiment of the present disclosure, the one or more health condition parameters are selected from the group including (or consisting of) a physiological parameter, a physical stress parameter indicative of a physical stress condition of the patient, a mental stress parameter indicative of a mental stress condition of the patient, and combinations thereof.

According to some embodiments, which can be combined with other embodiments described herein, the medical support system may be configured to conduct a dialogue with the patient, for example, by asking the patient one or more predetermined questions.

According to some embodiments, which can be combined with other embodiments described herein, the medical support system may be configured to identify the patient based on the received sound.

According to some embodiments, which can be combined with other embodiments described herein, the audio reception module may be selected from the group including (or consisting of) a microphone, a mobile terminal, a stationary device, a wearable, a smart device, an acceleration sensor, an implant, and combinations thereof.

According to some embodiments, which can be combined with other embodiments described herein, the medical support function may be selected from the group including (or consisting of) medical diagnostics, medical decision-making, medical treatment of the patient, and combinations thereof.

According to some embodiments, which can be combined with other embodiments described herein, the medical support system may be configured for chronic cardiac disease diagnostics and/or chronic cardiac disease treatment. Additionally, or alternatively, the medical support system may be configured for chronic respiratory disease diagnostics and/or chronic respiratory disease treatment. Additionally, or alternatively, the medical support system may be configured for chronic mental illness diagnostics and/or chronic mental illness treatment.

According to some embodiments, which can be combined with other embodiments described herein, the medical support system may further include at least one output device. The medical support system may be configured to control the at least one output device to output information indicative of the analysis of the sound data.

According to some embodiments, which can be combined with other embodiments described herein, the at least one output device may be selected from the group including (or consisting of) a display device and an acoustical device.

According to some embodiments, which can be combined with other embodiments described herein, the medical support system may further include a control module configured to control one or more medical treatment devices based on the analysis of at least the sound data.

According to some embodiments, which can be combined with other embodiments described herein, the control module may be configured to initiate and/or change a treatment of the patient by the control of the one or more medical treatment devices.

According to another independent aspect of the present disclosure, a medical support method for patient treatment is provided. The medical support method includes receiving sounds of a patient and generating sound data indicative of the received sounds; analysing, by an artificial intelligence module, the sound data; and providing a medical support function at least based on the analysis of the sound data.

Embodiments are also directed at systems for carrying out the disclosed methods and include system aspects for performing each described method aspect. These method aspects may be performed by way of hardware components, a computer programmed by appropriate software, by any combination of the two or in any other manner. Furthermore, embodiments according to the invention are also directed at methods for operating the described system. It includes method aspects for carrying out every function of the medical support system.

According to another independent aspect of the present disclosure, a machine-readable medium is provided. The machine-readable medium includes instructions executable by one or more processors to implement the medical support method for patient treatment of the embodiments of the present disclosure.

The (e.g. non-transitory) machine readable medium may include, for example, optical media such as CD-ROMs and digital video disks (DVDs), and semiconductor memory devices such as Electrically Programmable Read-Only Memory (EPROM), and Electrically Erasable Programmable Read-Only Memory (EEPROM). The machine-readable medium may be used to tangibly retain computer program instructions or code organized into one or more modules and written in any desired computer programming language. When executed by, for example, one or more processors such computer program code may implement one or more of the methods described herein.

According to another independent aspect of the present disclosure, a medical support system for patient treatment is provided. The medical support system includes one or more processors; and a memory (e.g., the above machine-readable medium) coupled to the one or more processors and comprising instructions executable by the one or more processors to implement the medical support method for patient treatment of the embodiments of the present disclosure.

So that the manner in which the above recited features of the present disclosure can be understood in detail, a more particular description of the disclosure, briefly summarized above, may be had by reference to embodiments. The accompanying drawings relate to embodiments of the disclosure and are described in the following:
Fig. 1 shows a schematic view of a medical support system for patient treatment according to embodiments described herein;
Fig. 2 shows a schematic view of a medical support system for patient treatment according to further embodiments described herein; and
Fig. 3 shows a flow chart of a medical support method for patient treatment according to embodiments described herein.

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

Nowadays it is increasingly difficult to find the optimal treatment for a patient. These difficulties are due, at least in part, to rising cost pressures, an increasing number of treatment options, and increased information availability.

The embodiments of the present disclosure address the above challenges by analyzing sounds, such as speech, of the patient in an artificial intelligence module to determine and/or monitor a condition of the patient. Analyzing the sounds or speech or voice of the patient enables an improved medical support function. For example, digital monitoring of chronic heart failure can be provided, allowing the needs of a large number of different patients to be addressed. Furthermore, identification of optimal treatments can be achieved. In addition, treatment time can be minimized, and medical costs reduced.

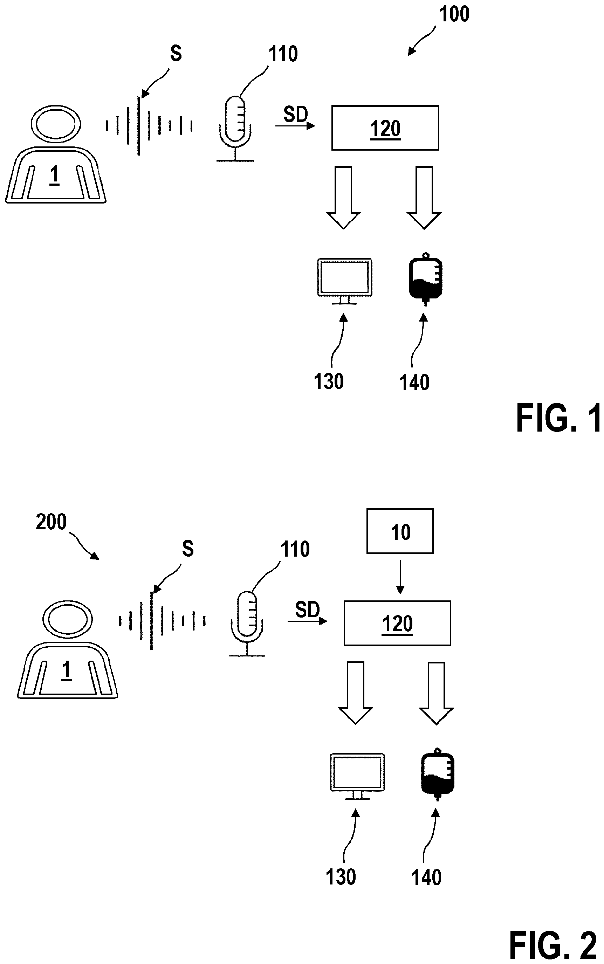

Fig. 1 shows a schematic view of a medical support system 100 for patient treatment according to embodiments described herein.

The medical support system 100 includes an audio reception module 110 configured to receive sounds S, such as speech, of a patient 1 and generate sound data SD indicative of the received sounds S; and an artificial intelligence module 120 configured to analyze the sound data SD, wherein the artificial intelligence module 120 is further configured to provide a medical support function for the patient 1 based on the analysis of the sound data SD.

In some embodiments, the medical support function is a medical diagnostics function, a medical decision-making function, a medical treatment function, or a combination thereof. For example, the medical support system 100 may be configured for chronic cardiac disease diagnostics and/or chronic cardiac disease treatment, chronic respiratory disease diagnostics and/or chronic respiratory disease treatment, or chronic mental illness diagnostics and/or chronic mental illness treatment. The medical support system 100 may use the information included in the patients' voice to provide improved diagnostics and/or treatment in respect to at least one of the aforementioned disease or illness.

The term "module" as used throughout the present application may be understood in the sense of software components and/or software instances which are designed to implement different tasks of the medical support system 100 of the present disclosure.

The term "artificial intelligence" as used throughout the present application may be understood in the sense of software components or software instances which are designed to correctly interpret data (i.e., the sound data SD and optionally further data), to learn from such data, and to use those learnings to provide a medical support function through flexible adaptation.

According to some embodiments of the present disclosure, the artificial intelligence module 120 may be configured to determine at least one biomarker in (or based on) the sound data SD and to provide the medical support function based on the determined at least one biomarker.

Generally, a biomarker or biological marker is a measurable indicator of a biological state or condition, e.g., related to at least one of the aforementioned diseases or illnesses, such as a chronic cardiac disease.

In a preferred embodiment the biomarker is a vocal biomarker. The vocal biomarker is a sound feature or voice feature indicative of at least one of the aforementioned diseases or illnesses, such as a cardiac disease. For example, the vocal biomarker may be selected from the group including (or consisting of) a speech rate, shortness of breath, suppression of sounds, and unclear pronunciation. However, the present disclosure is not limited thereto and the biomarker can be another sound feature or voice feature indicative of a specific disease or illness.

For the extraction of suitable vocal biomarkers, preferably machine learning is used to train a corresponding artificial intelligence model. According to the present disclosure, the artificial intelligence models can be used for specific indications, i.e., the artificial intelligence models are trained individually for different indications, such as chronic heart failure, depression, hypertension, chronic obstructive pulmonary disease (COPD, and the like.

In exemplary embodiments, a neural network can be implemented to analyse the sound data SD. A neural network is based on a collection of connected nodes. A node that receives a signal processes it and can signal nodes connected to it. Typically, nodes are aggregated into layers. Different layers may perform different transformations on their inputs. Signals travel from an input layer to an output layer. Such a neural network may be trained by processing examples, each of which contains a known input and result, forming probability-weighted associations between the two, which are stored within the data structure of the neural network. Thus, the neural network learns to perform tasks by considering examples.

Preferably, a deep learning architecture is used, i.e., a neural network having more than one hidden layer. One non-limiting example is a feed forward network with multiple hidden layers which are relatively easy to implement and train. Another non-limiting example is a convolutional neural network, which can be used preferentially for recognition of images, speech, and biosignals (i.e., multidimensional input vectors). Other non-limiting examples include recurrent neuronal networks and generative adversarial networks (GANs).

In some embodiments of the present disclosure, self-organizing maps, such as the Kohonen feature map, can be used for automated identification of vocal biomarkers, as they are quite robust for this application.

Preferably, the artificial intelligence module 120 is configured to perform input signal conditioning (e.g., filtering, scaling, normalization, transformation, and the like) and/or result post-processing (e.g., clustering, weighting, filtering, plausibility checking, and the like).

Additionally, or alternatively, an automated model verification and/or model health monitoring and/or model drift monitoring can be implemented in order to ensure the quality of the analysis result.

Additionally, or alternatively, a data and/or model governance layer can be implemented to make the medical application traceable and audit-proof (e.g. for documentation purposes).

According to some embodiments, the audio reception module 110 may be selected from the group including (or consisting of) a microphone, a mobile terminal, a stationary device, a wearable, a smart device, an acceleration sensor, an implant, and combinations thereof.

The term "mobile terminal" includes computer devices which are mobile (e.g., vehicles or mobility assistance devices) and/or portable (e.g., smartphones or wearables), and that are equipped with communication technology. Examples of mobile terminals include mobile telephones or smartphones, portable gaming devices, laptops, wearable devices (e.g., smart watches, smart glasses), PDAs, portable Internet devices, music players, data storage devices, or other handheld devices.

The stationary device may have network connection capabilities. For example, the stationary device may be a smart device, such as a smart speaker.

In further embodiments, the audio reception module 110 may be a medical implant. The implant may have a microphone and/or an acceleration sensor to detect the patient's voice.

In yet further embodiments, the audio reception module 110 may be comprised in a dedicated medical device or system, such as a dedicated speech acquisition system for medical applications.

According to some embodiments, which can be combined with other embodiments described herein, the medical support system 100 further includes a communication module (not shown) configured to receive the sound data SD from the audio reception module 110. This may be beneficial if the audio reception module 110 is an external device such as a smartphone of the patient 1.

For example, the communication module and the audio reception module 110 may communicate via at least one transmission medium, such as a network. In a preferred embodiment, the at least one transmission medium includes a mobile network and/or a local network.

The mobile network may use any of various wireless communication technologies, or telecommunication standards, such as GSM, UMTS, LTE, LTE-Advanced (LTE-A), 5G, HSPA, and the like. For example, the communication module of the medical support system 100 may include a communication profile such as an embedded subscriber identification module, eSIM, profile. However, the present disclosure is not limited thereto, and a conventional SIM may be used or another non-SIM communication profile.

The local network may use any of various wired and/or wireless communication technologies, such as Local Area Networks (LANs), Wireless LAN (WiFi), Bluetooth, and the like.

According to some embodiments, which can be combined with other embodiments described herein, the medical support system 100 may be configured to identify the patient 1 based on the received sound. In particular, the medical support system 100 may perform an unambiguous identification of the patient's voice to exclude confusion with other persons.

According to some embodiments, which can be combined with other embodiments described herein, the medical support system 100 may be configured to conduct a dialogue with the patient, for example, by asking the patient one or more predetermined questions. For example, the medical support system 100 may conduct a system-guided voice dialog with the patient 1 in the form of a brief anamnesis. The patient's anamnestic responses may then be evaluated AI-based by the artificial intelligence module 120.

According to some embodiments, which can be combined with other embodiments described herein, the medical support system 100 may be configured to perform the audio recording and/or analysis on a regular basis, such as one or more times per day.

According to some embodiments, which can be combined with other embodiments described herein, the medical support system 100 further includes at least one output device 130. The medical support system 100 may be configured to control the at least one output device 130 to output information indicative of the analysis of the sound data SD. For example, the at least one output device 130 may include at least one display device and/or at least acoustical device (e.g., at least one loudspeaker).

In some embodiments, the output information provided by the at least one output device 130 may include, or be, a medical diagnosis and/or medical treatment instructions. The output information may be, for example, instructions for the administration of a drug by medical personnel.

According to some embodiments, which can be combined with other embodiments described herein, the medical support system 100 further includes a control module (not shown) configured to control one or more medical treatment devices 140 based on the analysis of the sound data SD. An example of a medical treatment device is a device for drug administration or automatic drug administration.

In some implementations, the control module of the medical support system 100 is configured to initiate and/or change a treatment of the patient 1 by the control of the one or more medical treatment devices 140. For example, the control module may change a drug dose administered to the patient 1 based on the analysis of the sound data SD.

Fig. 2 shows a schematic view of a medical support system 200 for patient treatment according to further embodiments described herein. The medical support system 200 is similar to the medical support system of FIG. 1 and therefore, a description of identical or similar features is not repeated.

According to some embodiments, which can be combined with other embodiments described herein, the medical support system 100 includes or is (e.g., wired or wirelessly) connected to one or more sensors 10 associated with the patient 1. The one or more sensors 10 may be configured to receive or detect one or more health condition parameters of the patient 1 and provide corresponding condition data to the artificial intelligence module 120. The artificial intelligence module 120 may then provide the medical support function based on both the sound data SD and the one or more health condition parameters.

For example, a patient with a chronic disease such as heart failure can be regularly monitored with respect to a physiological parameter, such as an ECG recording. In addition, the patient's voice is regularly recorded and both pieces of information are forwarded to the artificial intelligence module 120 where they are analyzed, e.g., with regard to the progression of this chronic disease. If necessary, appropriate medical measures (e.g., information to a doctor, the patient, relatives and/or a treatment) can be initiated automatically.

The one or more sensors 10 can be configured for non-invasive and/or invasive monitoring. For example, the one or more sensors 10 can be connected or attached to the patient's body to determine or monitor the one or more health condition parameters in an invasive or a non-invasive manner.

Preferably, the one or more health condition parameters are selected from the group including (or consisting of) a physiological parameter, a physical stress parameter indicative of a physical stress condition of the patient, a mental stress parameter indicative of a mental stress condition of the patient, and combinations thereof.

In some embodiments of the present disclosure, the physiological parameter can be selected from the group including (or consisting of) heart rate, blood pressure, body temperature, and serum levels (e.g., of various stress hormones). However, the present disclosure is not limited thereto, and other physiological parameters useful in determining a health condition of the patient 1 may be measured by the one or more sensors 10.

Preferably, the physical stress parameter and/or the mental stress parameter may be determined using an impedance-based technology.

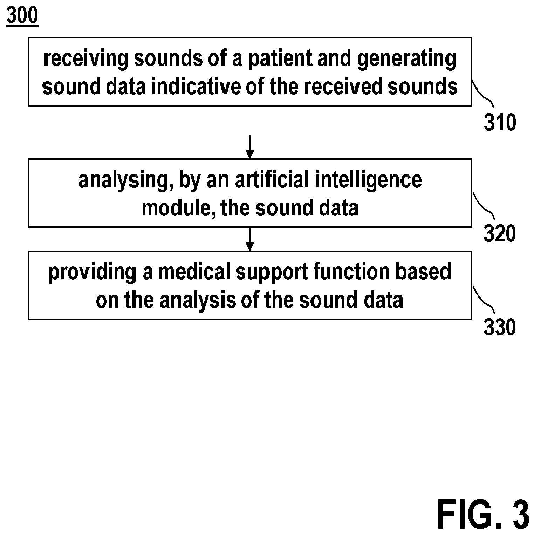

Fig. 3 shows a flow chart of a medical support method 300 for patient treatment according to embodiments described herein.

The medical support method 300 includes in block 310 receiving sounds, such as speech, of a patient and generating sound data indicative of the sounds received from the patient; in block 320 analysing, by an artificial intelligence module, the sound data; and in block 330 providing a medical support function for the patient based on the analysis of the sound data.

According to embodiments described herein, the medical support method for patient treatment can be conducted by means of computer programs, software, computer software products and the interrelated controllers, which can have a CPU, a memory, a user interface, and input and output means being in communication with the corresponding components of the medical support system for patient treatment.

Embodiments of the present disclosure analyze sounds, such as speech, of the patient in an artificial intelligence module to determine and/or monitor a condition of the patient. Analyzing the sounds or speech or voice of the patient enables an improved medical support function. For example, digital monitoring of chronic heart failure can be provided, allowing the needs of a large number of different patients to be addressed. Furthermore, identification of optimal treatments can be achieved. In addition, treatment time can be minimized, and medical costs reduced.

While the foregoing is directed to embodiments of the disclosure, other and further embodiments of the disclosure may be devised without departing from the basic scope thereof, and the scope thereof is determined by the claims that follow.

## Claims

1. A medical support system (100) for patient treatment, comprising:
an audio reception module (110) configured to receive sounds (S) of a patient (1) and generate sound data (SD) indicative of the received sounds (S); and
an artificial intelligence module (120) configured to analyze the sound data (SD), wherein the artificial intelligence module (120) is further configured to provide a medical support function based on the analysis of the sound data (SD).

2. The medical support system (100) of claim 1, wherein the artificial intelligence module (120) is configured to implement a machine learning algorithm, in particular a neural network, to analyse the sound data (SD).

3. The medical support system (100) of claim 1 or 2, wherein the artificial intelligence module (120) is configured to determine at least one vocal biomarker in the sound data (SD) and to provide the medical support function based on the determined at least one vocal biomarker.

4. The medical support system (100) of any one of the preceding claims, wherein the artificial intelligence module (120) is configured to provide the medical support function further based on one or more health condition parameters of the patient (1), and wherein the one or more health condition parameters are selected from the group consisting of a physiological parameter, a physical stress parameter indicative of a physical stress condition of the patient, a mental stress parameter indicative of a mental stress condition of the patient, and combinations thereof.

5. The medical support system (100) of any one of the preceding claims, wherein the medical support system (100) is configured to conduct a dialogue with the patient (1), in particular by asking predetermined questions.

6. The medical support system (100) of any one of the preceding claims, wherein the medical support system (100) is configured to identify the patient (1) based on the received sound (S).

7. The medical support system (100) of any one of the preceding claims, wherein the audio reception module (110) is selected from the group consisting of a microphone, a mobile terminal, a wearable, a stationary device, a smart device, an acceleration sensor, an implant, and combinations thereof.

8. The medical support system (100) of any one of the preceding claims, wherein the medical support function is selected from the group consisting of medical diagnostics, medical decision-making, medical treatment of the patient (1), and combinations thereof.

9. The medical support system (100) of any one of the preceding claims, wherein the medical support system (100) is configured for at least one of:
chronic cardiac disease diagnostics and/or chronic cardiac disease treatment;
chronic respiratory disease diagnostics and/or chronic respiratory disease treatment; and
chronic mental illness diagnostics and/or chronic mental illness treatment.

10. The medical support system (100) of any one of the preceding claims, further including at least one output device (130), wherein the medical support system (100) is configured to control the at least one output device (130) to output information indicative of the analysis of the sound data (SD).

11. The medical support system (100) of claim 10, wherein the at least one output device (130) is selected from the group consisting of a display device and an acoustical device.

12. The medical support system (100) of any one of the preceding claims, further comprising a control module configured to control one or more medical treatment devices (140) based on the analysis of the sound data (SD).

13. The medical support system (100) of claim 12, wherein the control module is configured to initiate and/or change a treatment of the patient (1) by the control of the one or more medical treatment devices (140).

14. Medical support method (300) for patient treatment, comprising:
receiving (310) sounds of a patient and generating sound data indicative of the received sounds;
analysing (320), by an artificial intelligence module, the sound data; and
providing (330) a medical support function based on the analysis of the sound data.

15. A machine readable medium comprising instructions executable by one or more processors to implement the medical support method (300) according to claim 14.
